# EUROPÄISCHE PATENTSCHRIFT

(11) **EP 4 444 227 B1**
(45) Veröffentlichungstag und Bekanntmachung des Hinweises auf die Patenterteilung: **24.09.2025**
(21) Anmeldenummer: 22829728.9
(22) Anmeldetag: 01.12.2022
(51) Int. Cl.: A61F 2/58

(54) **PROTHESENFINGER UND PROTHESENHAND MIT PROTHESENFINGER**
PROSTHETIC FINGER AND PROSTHETIC HAND HAVING A PROSTHETIC FINGER
DOIGT PROTHÉTIQUE ET MAIN PROTHÉTIQUE AYANT UN DOIGT PROTHÉTIQUE

(30) Priorität: 08.12.2021 DE 102021132277
(43) Veröffentlichungstag der Anmeldung: 16.10.2024
(73) Patentinhaber: Otto Bock Healthcare Products GmbH, 1110 Wien (AT)
(72) Erfinder: EDER, Florian, 1110 Wien (AT); GÜRTL, Josef, 1110 Wien (AT); LEDINGER, Christoph, 1110 Wien (AT)
(74) Vertreter: Gramm, Lins & Partner Patent- und Rechtsanwälte PartGmbB
(86) Internationale Anmeldenummer: PCT/EP2022/084047
(87) Internationale Veröffentlichungsnummer: WO 2023/104630

(56) Entgegenhaltungen:
- EP-A1- 2 653 137
- DE-A1- 102005 061 313
- US-A1- 2016 235 554

## Beschreibung

Die Erfindung betrifft einen Prothesenfinger, dem ein motorischer Antrieb zugeordnet ist, über den der Prothesen Finger relativ zu einem Chassis um eine erste Schwenkachse verschwenkbar ist, mit einem Fingerelement, das um die erste Schwenkachse verschwenkbar an dem Chassis gelagert und mit einem Träger gekoppelt ist, der Träger ist mit einem Antriebselement gekoppelt, das mit dem Antrieb koppelbar ist und drehmomentübertragend mit dem Träger gekoppelt ist. Die Erfindung betrifft ebenfalls eine Prothesenhand mit einen Prothesenfinger, der an einem Chassis der Prothesenhand gelagert ist.

Prothesenfinger und Prothesenhände ersetzen nicht oder nicht mehr vorhandene Finger oder Hände. Sowohl die Prothesenfinger als auch die Prothesenhände bilden die menschliche Anatomie nach. Prothesenhände weisen in der Regel vier Prothesenfinger auf, die um im Wesentlichen parallel zueinander orientierten Schwenkachsen in dem Verbindungsbereich zu dem Chassis verschwenkbar gelagert sind. Die Prothesenfinger weisen gegebenenfalls weitere, im Wesentlichen parallel dazu orientierte, distale Schwenkachsen an den Fingerelementen auf, um eine Faust bilden zu können. Prothesenhände weisen ebenfalls einen Daumen auf, der ebenfalls als ein Finger gilt. Der Daumen ist ein wesentlicher Bestandteil der menschlichen Hand uns macht diese zu einem Universalwerkzeug, das eine Vielzahl von Bewegungsmöglichkeiten und Freiheitsgraden aufweist. Die Bedeutung des Daumens liegt insbesondere darin, dass seine relativ zu den übrigen Fingern eingenommene Position maßgeblich für die unterschiedlichen Griffbilder ist. Wichtige Positionen und Stellungen sind der Oppositionsgriff und der Lateralgriff. **In** dem

Oppositionsgriff können große Gegenstände gehalten und kleinere Gegenstände gegriffen werden. Der Lateralgriff ist vor allem für flache Gegenstände hilfreich, wird aber auch beim flachen Auflegen der Hand auf einen Tisch, bei dem Greifen einer Computermaus und dergleichen verwendet.

Das Daumengrundgelenk einer natürlichen Hand ist in Gestalt eines Sattelgelenkes ausgebildet. Bei Prothesenhänden werden die damit erreichbaren Positionen häufig über eine Kombination von Drehgelenken versucht zu realisieren, die der Daumenbasis um winkelig zueinander orientierte Schwenkachsen eine Verschwenkung ermöglichen. Ein erstes Drehgelenk um eine erste Schwenkachse steuert die Flexion und Extension des Fingerelementes des Daumens, wodurch Gegenstände gegriffen und losgelassen werden. Dabei wird das Fingerelement in palmarer Richtung bewegt. Das andere Gelenk ermöglicht eine Abduktion und Adduktion des Daumens, wodurch unterschiedliche Griffarten wie der Oppositionsgriff und der Lateralgriff ermöglicht werden. Das Fingerelement des Daumens wird dabei in die jeweilige Oppositionsposition bzw. Lateralposition verfahren.

Aktive Bewegungen von Prothesen werden häufig über myoelektrische Signale gesteuert und veranlasst. Dabei werden über Elektroden von in Muskelzellen erzeugte elektrische Signale detektiert und einer Steuerungseinrichtung übermittelt, die nach einer Verarbeitung der Signale und gegebenenfalls einer Verstärkung einen motorischen Antrieb aktiviert oder deaktiviert. Die Energie für die Verstellung wird über einen Energiespeicher, insbesondere einen Akkumulator bereitgestellt. Um ausreichend große Kräfte und Geschwindigkeiten zu erzielen, ist der motorische Antrieb mit dem Prothesenfinger gegebenenfalls über nachgeschaltete Getriebestufen gekoppelt. Jede Bewegung eines Prothesenfingers verbraucht Energie, die aus dem Energiespeicher entnommen werden muss, wobei nur ein begrenzter Energievorrat in dem Energiespeicher vorhanden ist. Eine Prothesenhand mit einem Antrieb ist beispielsweise in der EP 2 125 091 B1 beschrieben

Eine passive Bewegung des Prothesenfingers oder einer Prothesenhand liegt dann vor, wenn durch Einwirkung von externen Kräften die Prothesenfinger relativ zu dem Chassis bzw. die Prothesenhand als solche bewegt wird. Solche Bewegungen werden beispielsweise bewusst durch die kontralaterale Hand des Prothesenträgers oder durch das Aufbringen von Druckkräften bzw. Zugkräften bei der Benutzung der Prothesenhand bewirkt. Passive Bewegungen verbrauchen keine Energie aus dem Energiespeicher. Sofern die Prothesenhand oder ein Prothesenfinger elastisch gelagert ist, erfolgt eine Rückstellung in eine Ausgangsposition nach dem Nachlassen einer externen Kraft. Durch Rastelemente ist es möglich, dass nach einer passiven Bewegung der Prothesenfinger oder die Prothesenhand in einer bestimmten Position beibehalten wird, beispielsweise um einen Prothesenfinger in einer angelegten oder abgespreizten Stellung zu halten.

Die US 2016 / 0 250 044 A1 betrifft einen Zahnradverriegelungsmechanismus für eine Handprothese mit einem ersten Innenzahnrad und einem zweiten Innenzahnrad. Das erste Innenzahnrad ist an einem distalen Fingerglied eines Fingerelementes befestigt. Das zweite Innenzahnrad ist an einem proximalen Fingerglied des Fingerelementes befestigt. Ein Außenzahnrad ist mit einem Knopf gekoppelt und bildet eine Brücke zwischen dem ersten Innenzahnrad und dem zweiten Innenzahnrad, um ein Gelenk des Fingerelementes zu verriegeln. Eine Feder ist so ausgebildet, dass sie den Knopf in eine verriegelte Stellung zurückbringt.

Die DE 10 2008 056 520 A1 betrifft ein Fingerelement mit einer Trägerkomponente, einem ersten Fingerglied mit einer ersten Gelenkverbindung zur Trägerkomponente und einem zweiten Fingerglied mit einer zweiten Gelenkverbindung zum ersten Fingerglied. Zwischen der ersten und der zweiten Gelenkverbindung ist ein Kopplungsmechanismus angeordnet. Ein Stellantrieb für die erste Gelenkverbindung weist einen Motor mit Antriebswelle und einem Schneckengetriebe mit einer Gewindeschnecke und mit einem auf die Gewindeschnecke eingreifenden Zahnsegment auf. Die Gewindeschnecke ist auf der Antriebswelle axial bewegbar formschlüssig gelagert und axial durch separate Führungen geführt.

Die US 2016/235554 A1 betrifft ein Handprothesensystem mit mehreren Fingerprothesen und einer Daumenprothese. Das Handprothesensystem weist einen Daumenantriebsmechanismus zum Betätigen des Prothesendaumens auf, der so konfiguriert ist, dass der Prothesendaumen eine Klemm- oder Greifbewegung und eine Loslassbewegung ausführen kann. Das Handprothesensystem weist außerdem eine Verriegelung auf, um den Klemm- oder Greifdruck aufrechtzuerhalten, nachdem ein Motor ausgeschaltet wurde. Über eine Zahnradsperre kann ein Fingergelenk gesperrt werden. Das Prothesenhandsystem kann außerdem ein adaptives Greifgelenk umfassen, das sich an jedem Prothesenfinger befinden kann. Das adaptive Greifgelenk ist so konfiguriert, dass es die Vielzahl der Prothesenfinger passiv an ein oder mehrere unterschiedlich geformte Objekte anpasst.

Die DE10 2005 061 313 A1 betrifft eine Handprothese mit einem Chassis, an dem mehrere Fingerprothesen gelenkig gelagert sind, die über einen Antrieb um zumindest eine Schwenkachse relativ zu dem Chassis und aufeinander zu bewegbar sind. Die Kraftübertragungseinrichtungen sind an einem gemeinsamen Antrieb dergestalt mit den Fingerprothesen gekoppelt, dass, ausgehend von einer Ruhestellung in Abhängigkeit von der Drehrichtung des Antriebes, zumindest zwei Fingerprothesen unterschiedliche Verstellwinkel relativ zu dem Chassis durchlaufen.

Die EP 2 653 137 A1 betrifft eine Handgelenkprothese mit einer Mittelhand und einem daran befestigten Daumen sowie Zeigefinger, der um eine Schwenkachse bewegt werden kann. Der Daumen kann um eine Adduktionsachse gedreht werden, indem ein Elektromotor, der in die Prothese integriert ist, Übertragungsmittel antreibt. Ebenfalls wird ein rotierendes Hauptelement über den Elektromotor betätigt. Die Übertragungsmittel sind ausgebildet, um den Daumen und den Zeigefinger im Wesentlichen selektiv zu steuern. Die Übertragungsmittel sind so ausgebildet, dass eine erste Winkelbewegung des rotierenden Hauptelements eine deutliche Drehung des Zeigefingers zwischen einer geschlossenen und einer offenen Position erzeugt, während der Daumen während der ersten Winkelbewegung blockiert bleiben.

Aufgabe der vorliegenden Erfindung ist es, einen Prothesenfinger und eine Prothesenhand bereitzustellen, die länger eingesetzt werden können, ohne dass ein Energiespeicher aufgeladen werden muss.

Diese Aufgabe wird durch einen Prothesenfinger bzw. eine Prothesenhand mit den Merkmalen der unabhängigen Ansprüche gelöst. Vorteilhafte Ausgestaltungen und Weiterbildungen der Erfindung sind in den Unteransprüchen, der Beschreibung sowie den Figuren offenbart.

Der Prothesenfinger, dem ein motorische Antrieb zugeordnet ist, über den der Prothesenfinger relativ zu einem Chassis um eine erste Schwenkachse verschwenkbar ist, mit einem Fingerelement, das um die erste Schwenkachse verschwenkbar an dem Chassis gelagert und mit einem Träger gekoppelt ist, wobei der Träger mit einem Antriebselement gekoppelt ist, das mit dem Antrieb koppelbar und drehmomentübertragend mit dem Träger gekoppelt ist, sieht vor, dass das Antriebselement über ein Kopplungselement mit dem Träger drehmomentübertragend gekoppelt ist, wobei das Kopplungselement axial verschieblich und in Richtung auf das Antriebselement elastisch vorgespannt an dem Träger gelagert ist. Der Prothesenfinger ist insbesondere als ein Prothesendaumen ausgebildet, wobei das Fingerelement mehrgliedrig, insbesondere zweigliedrig ausgebildet sein kann, sodass eine distale Komponente, analog zu einem natürlichen Finger oder Daumen, verschwenkbar an einer proximalen Komponente gelagert ist. Die erste Schwenkachse ermöglicht bei der Anwendung bei einem Daumen insbesondere eine Abduktion und Adduktion und damit eine Verdrehung des Fingerelementes um eine Schwenkachse, die im Wesentlichen in Längserstreckung der Prothesenhand orientiert ist und sich von einem Handgelenk in Richtung zu den Fingerspitzen erstreckt. Bei einer Verwendung für einen Finger erstreckt sich die erste Schwenkachse vorzugsweise senkrecht zu der Längserstreckung des Fingerelementes, also senkrecht zu der proximal-distal-Orientierung. Das Fingerelement ist mit einem Träger gekoppelt und kann direkt an dem Träger gelagert sein, beispielsweise schwenkbar daran oder über ein Getriebe oder ein Kopplungselement mit dem Träger gekoppelt sein, so dass die schwenkbare Lagerung über eine Lagerstelle beispielsweise an dem Chassis verwirklicht wird. Der Träger selbst ist mit einem Antriebselement gekoppelt, insbesondere ist das Antriebselement an dem Träger gelagert und befestigt, wobei das Antriebselement mit dem Antrieb koppelbar und im zusammengefügten Zustand der Prothesenhand gekoppelt ist. Der Antrieb als insbesondere elektromotorischer Antrieb steht in Eingriff mit dem Antriebselement und treibt dieses an, wobei das Antriebselement wiederum dergestalt mit dem Träger gekoppelt ist, dass die Kraft oder das Drehmoment von dem Antrieb auf den Träger übertragen wird. Die Kopplung erfolgt dabei über ein Kopplungselement, das wiederum mit dem Träger drehmomentübertragend gekoppelt ist, insbesondere direkt formschlüssig oder über ein oder mehrere Zwischenelemente mit dem Träger verbunden oder gekoppelt ist. Das Kopplungselement ist dabei axial verschieblich entlang der Längserstreckung des Trägers an dem Träger gelagert und ist in Richtung auf das Antriebselement elastisch vorgespannt. Dadurch ist es möglich, dass der Träger und damit der gesamte Prothesenfinger neben einer aktiven Verstellung durch den Antrieb auch passiv durch einen Nutzer oder während der Benutzung durch extern wirkende Kräfte verstellt werden kann. Insbesondere sind dann eine Abduktion und Adduktion bei entsprechender Orientierung und eine Verschwenkung um die erste Schwenkachse sowohl aktiv als auch passiv möglich. Über die elastische Vorspannung in Richtung auf das Antriebselement beeinflusst das Kopplungselement nicht die aktive Verstellung durch den Antrieb, sodass bei einer passiven Verstellung keine Energie, insbesondere keine gespeicherte elektrische Energie verbraucht wird. Gleichzeitig wird bei einer aktiven Verstellung durch den motorischen Antrieb keine Überwindung einer Haltekraft oder einer Rückstellkraft aufgrund eines elastischen Elementes notwendig, sodass der aktive Betrieb des Prothesenfingers hinsichtlich des Energieverbrauchs optimiert erfolgen kann.

**In** einer Ausgestaltung ist das Antriebselement als Zahnrad oder Zahnradsegment ausgebildet und weist stirnseitig, also nicht an dem Umfang des Zahnrades oder Zahnradesegmentes, Rastelemente und/oder Reibbereiche auf. Das Kopplungselement weist ebenfalls Rastelemente und/oder Reibbereiche auf und ist als Rastscheibe ausgebildet, wobei die Rastelemente und/oder Reibbereiche korrespondierend zu den stirnseitigen Rastelementen und/oder Reibbereichen des Antriebselementes ausgebildet sind. Durch die Ausgestaltung des Antriebselementes und des Kopplungselementes als Rastscheiben werden definierte Raststellungen des Antriebselementes relativ zu dem Kopplungselement und damit auch definierte Stellungen des Prothesenfingers relativ zu dem Chassis ermöglicht. Die Positionierung über Rastelemente beeinflusst den aktiven Antriebsstrang mit dem motorischen Antrieb nicht, solange ein einstellbares Widerstandsmoment nicht überschritten wird. Das Widerstandsmoment ergibt sich aus der elastischen Vorspannung des Kopplungselementes in Richtung auf das Antriebselement sowie den geometrischen Ausgestaltungen der Rastelemente bzw. den Reibeigenschaften der Reibbereiche. Aufgrund der Verrastung und der bevorzugt in Umfangsrichtung gleichmäßig beabstandeten Anordnung von Rastelementen kann der Nutzer den Prothesenfinger, insbesondere den Daumen, passiv rasch verschwenken oder zur Seite schwenken, beispielsweise um die Prothesenhand flach auf den Tisch zu legen.

**In** einer Ausgestaltung sind die Rastelemente an dem Antriebselement sowie die korrespondierend ausgebildeten Rastelemente an dem Kopplungselement als Planverzahnung mit schraubenflächenförmigen Zahnflanken ausgeführt. Durch die Ausgestaltung der Zahnflanken in Form von Schraubenflächen wird ein Flächenkontakt über den gesamten Rastvorgang ermöglicht. Dadurch können die maximalen Flächenpressungen in dem Kontaktbereich zwischen den Zahnflanken reduziert werden, wodurch der gesamte konstruktive Aufbau bei gleicher Festigkeit oder bei gleichen übertragbaren Momenten deutlich reduziert werden kann.

Um die passive Verstellung zu erleichtern, sind die Rastelemente in zumindest eine Umfangsrichtung geneigt oder abgeschrägt ausgebildet, vorzugsweise sind die Rastelemente in beiden Umfangsrichtungen abgeschrägt oder geneigt ausgebildet. Die Abschrägungen oder Schrägen können geradlinig oder gekrümmt ausgebildet sein, sodass sich Zähne oder Wellen an den jeweiligen Stirnseiten des Antriebselementes und des Kopplungselementes ausbilden.

Das Antriebselement und das Kopplungselement sind bevorzugt mit einer Kronenverzahnung versehen und zumindest in Teilbereichen rotationssymmetrisch aufgebaut. Der Träger weist beispielsweise einen Zapfen auf, an dem das Kopplungselement formschlüssig und drehmomentübertragend gelagert ist. Dazu ist an dem Träger an seiner Außenseite eine Verzahnung oder eine andere Formschlusseinrichtung angeordnet oder ausgebildet, mit der eine axial Verschieblichkeit des Kopplungselementes gewährleistet ist und gleichzeitig ein Drehmoment übertragen werden kann, sodass der Träger um die erste Schwenkachse verschwenkt werden kann.

Der Träger ist verschwenkbar um die erste Schwenkachse gelagert, wobei das Fingerelement drehfest hinsichtlich der Achse mit dem Träger gekoppelt ist. Dadurch führt eine Verdrehung des Trägers um die Achse zu einer Verdrehung des Federelementes um entweder diese Achse oder um die Schwenkachse des Fingerelementes.

In einer Ausgestaltung ist das Fingerelement verschwenkbar an dem Träger gelagert, was insbesondere dann vorteilhaft ist, wenn der Träger selbst relativ zu dem Chassis verschwenkbar ist. Dadurch können die Fingerelemente oder Prothesenfinger besser und vielfältiger positioniert werden, um unterschiedliche Griffarten verwirklichen zu können und Gegenstände besser zugreifen.

In einer Ausgestaltung ist das Fingerelement verschwenkbar an dem Träger um die erste Schwenkachse gelagert, wodurch bewirkt wird, dass eine passive Bewegung um die erste Schwenkachse über das elastisch vorgespannte Kopplungselement ermöglicht wird. Die Verschwenkung um die zweite Schwenkachse kann ebenfalls mit einer Möglichkeit zur passiven Verschwenkung ausgestattet sein.

Das Fingerelement ist in einer Ausgestaltung über ein Zahnrad oder ein Zahnradsegment mit dem Träger gekoppelt, sodass gleichzeitig eine Getriebeübersetzung ausgebildet wird. Bestandteil des Getriebes ist dabei das Antriebselement, das auf den Träger einwirkt. Ein Drehmoment oder eine Drehbewegung wird über ein Zahnrad, das an dem Träger angeordnet oder

ausgebildet ist, auf ein Zahnradsegment oder ein vollständiges Zahnrad übertragen, das an dem Fingerelement angeordnet, ausgebildet oder daran befestigt ist.. Es können auch weitere Getriebestufen oder Zahnradstufen zwischen dem Träger und dem Fingerelement angeordnet oder ausgebildet sein, um eine entsprechende Drehbewegung des Trägers auf das Fingerelement zu übertragen.

Die elastische Vorspannung des Kopplungselementes in Richtung auf das Antriebselement erfolgt in einer Ausgestaltung über eine Feder, ein Elastomerelement und/oder eine Tellerfeder oder eine Tellerfederkombination.

**In** einer Ausgestaltung ist das Fingerelement an einem Halter schwenkbar um eine zweite Schwenkachse gelagert, wobei der Halter an dem Träger befestigt ist. Zusammen mit dem Träger verschwenkt der Halter und damit auch das Fingerelement, wenn der Träger um die erste Achse verschwenkt wird. Das Fingerelement kann unabhängig von einer Verschwenkung um die erste Achse um die zweite Schwenkachse verlagert werden, entweder passiv oder in einer Ausgestaltung aktiv. Dazu ist ein Antrieb vorgesehen, der in oder an dem Fingerelement gelagert ist und über den das Fingerelement motorisch verschwenkbar um die zweite Schwenkachse an dem Halter gelagert ist.

Die erste Schwenkachse und die zweite Schwenkachse sind nicht parallel und nicht kollinear zueinander ausgerichtet, sondern kreuzend, wobei sich die Schwenkachsen nicht schneiden müssen. Die Schwenkachsen können auch windschief zueinander und sich nicht kreuzend ausgerichtet sein.

Das Antriebselement ist in einer Ausgestaltung frei drehbar an dem Träger gelagert und bewirkt lediglich die Verdrehung des Kopplungselementes und dadurch eine Kraftübertragung auf den Träger, wenn das Kopplungselement mit einer ausreichenden Anpresskraft an dem Antriebselement anliegt und Drehmomente formschlüssig und/oder reibschlüssig überträgt.

Das Kopplungselement wird in Richtung auf das Antriebselement mit einer Vorspannkraft belastet, die ausreicht, um ein Drehmoment auf den Träger zu übertragen. Die Übertragung des Drehmomentes erfolgt aber nur bis zu einer gewissen, vorher festgelegten und vorteilhafterweise einstellbaren Belastungsgrenze. Wird der Widerstand gegen eine Verlagerung zu groß oder wird das aufgebrachte Drehmoment zu groß, entkoppelt der Träger sich von dem Kopplungselement dadurch, dass es relativ zu dem Träger axial verschoben wird. Dadurch sind einerseits ein Überlastschutz und andererseits eine passive Verstellbarkeit gegeben.

In einer Ausgestaltung ist vorgesehen, dass dem Träger ein Positionssensor oder ein Marker zugeordnet ist, über den die Stellung des Trägers relativ zu dem Chassis erfasst wird. Die Information über die Stellung des Trägers und damit auch des Prothesenfingers relativ zu dem Chassis ist wichtig, um bei einem nächsten Verstellbefehl durch den Nutzer über myoelektrische Signale zu wissen, in welche Ausgangsposition sich der Prothesenfinger befindet, damit daraus der notwendige Verstellweg berechnet wird. Wird der Prothesenfinger beispielsweise passiv verstellt und befindet sich in einer Rastposition, steht der Finger bei einem Schließbefehl nicht in der richtigen Stellung und kann über den Antrieb nicht in die gewünschte oder notwendige Stellung gefahren werden. Durch den Positionssensor werden Fehlbedienungen vermieden. Erzeugt der Nutzer über eine Kombination von Muskelkontraktionen ein bestimmtes Schließsignal, mit dem die Prothesenhand in eine bestimmte Endstellung, zum Beispiel Lateralgriff, gebracht werden soll, wird in der Steuerung ausgehend von den vorhandenen Positionssignalen der jeweils notwendige Verstellweg für den jeweiligen Prothesenfinger errechnet. Dadurch wird sichergestellt, dass unabhängig von der aktuellen Stellung des Prothesenfingers relativ zu dem Chassis bei jedem Befehl eine korrekte Endstellung erreicht wird.

Der Antrieb ist insbesondere als Elektromotor ausgebildet, der wiederum ein Abtriebselement, zum Beispiel ein Zahnrad oder eine Schnecke antreibt, das wiederum mit dem Antriebselement in einem montierten Zustand der Prothesenhand in Eingriff steht.

Der Antrieb kann mit dem Abtriebselement über ein Getriebe und/oder eine Kupplung gekoppelt sein. Der Antriebstrang von dem motorischen Antrieb, der insbesondere in dem Chassis gelagert ist, bis zu dem Kopplungselement ist in vorteilhafter Weise selbsthemmend ausgebildet, sodass bei einer Belastung des Prothesenfingers und/oder des Trägers keine Kräfte auf den Antrieb durchgeleitet werden. Die Selbsthemmung stellt zudem sicher, dass keine Energie zur Beibehaltung der einmal eingenommenen Stellung aufgewendet werden muss.

Neben der Möglichkeit, ohne den Einsatz elektrischer Energie eine einfache und schnell Verstellung in verschiedene Rastposition vorzunehmen, ermöglicht die beschriebene Ausgestaltung auch einen Überlastschutz für den Motor, falls eine unvorhergesehene Blockierung der Verstellung des Fingerelementes durch ein Hindernis oder dergleichen erfolgt.

Nachfolgend wird ein Ausführungsbeispiel der Erfindung anhand der Figuren näher erläutert. Welche Bezugszeichen bezeichnen gleiche Komponenten. Es zeigen:
Figur 1 - eine perspektivische Ansicht einer Prothesenhand;
Figur 2 - eine schematische Detaildarstellung des Antriebs;
Figur 3 - eine Detailansicht aus anderer Perspektive;
Figur 4 - eine Explosionsdarstellung eines Teils des Prothesenfingers;
Figur 5 - eine Schnittdarstellung durch eine Anordnung erhältlich Figur 3;
Figur 6 - eine Schnittdarstellung durch einen Prothesenfinger
Figur 7 - eine Explosion Darstellung einer Variante;
Figur 8 - eine Detailschnittdarstellung der Figur 7;
Figur 9 - eine perspektivische Detailansicht der Figur 7;
Figur 10 - eine Schnittdarstellung eines Details der Figur 8; sowie
Figur 11 - eine Explosionsdarstellung von Komponenten die Figur 10.

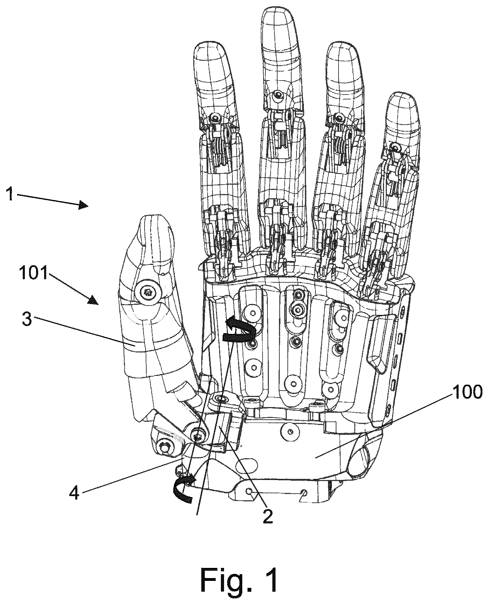

**In** der Figur 1 ist eine Prothesenhand 1 mit einem Chassis 100 und einem Prothesenfinger 101 dargestellt. Der Prothesenfinger 101 ist als Prothesendaumen ausgestaltet. An dem Chassis 100 sind darüber hinaus 4 weitere Prothesenfinger gelenkig und angetrieben befestigt. Die Prothesenfinger weisen ein Grundgelenk auf, das eine proximale Fingerkomponente mit dem Chassis gelenkig verbindet. An der proximalen Fingerkomponente ist eine distale Fingerkomponente gelenkig angeordnet, die entweder einzeln oder in Kombination mit der proximalen Fingerkomponente verlagert wird. Über nicht näher bezeichnete Antriebe innerhalb des Chassis kann jeder Finger einzeln oder können alle Finger gemeinsam bewegt werden, um eine Flexion in Richtung auf die Handinnenfläche oder eine Extension in die dargestellte, gestreckte Position auszuführen. Der Prothesenfinger 101 als Prothesendaumen ist um zwei Achsen 2, 4 schwenkbar relativ zu dem Chassis verlagerbar. Eine erste Achse 2, die später näher erläutert wird, ermöglicht eine Abduktion sowie eine Adduktion, die zweite Achse 4 ermöglicht eine Flexion in Richtung auf die Handinnenfläche oder die anderen Prothesenfinger. Auch der Prothesendaumen weist ein 2-gliedriges Fingerelement 3 mit einer proximalen

Fingerkomponente und einer distalen Fingerkomponente auf. Die distale Fingerkomponente ist, ähnlich wie zu den anderen Prothesenfinger beschrieben, schwenkbar an der proximalen Fingerkomponente gelagert. Ein Antrieb, der später näher erläutert wird, ist innerhalb des Chassis 100 gelagert und als Elektromotor ausgebildet. Ein Energiespeicher und einer Steuerungseinrichtung sind entweder innerhalb der Prothesenhand 1 angeordnet oder befinden sich in proximalen Prothesenkomponenten, beispielsweise einem Unterarm schaft.

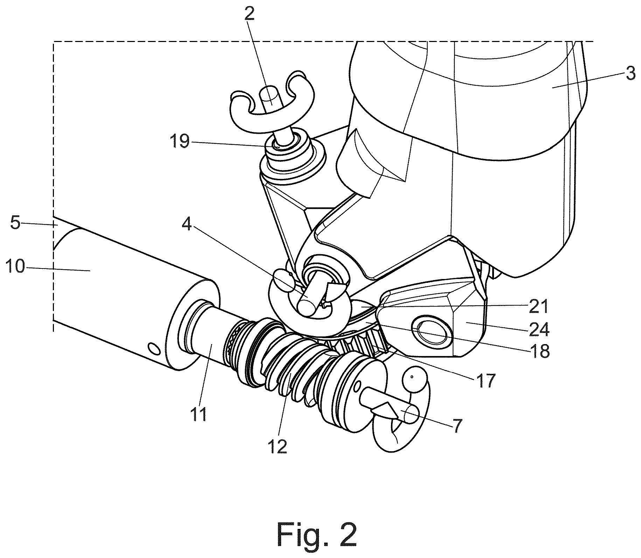

In der Figur 2 ist das Fingerelement 3 teilweise dargestellt, nämlich die proximale Fingerkomponente, die schwenkbar um die erste Schwenkachse 2 gelagert ist. Verschwenkung um die erste Schwenkachse 2 erfolgt über einen Halter 24, an dem das Fingerelement 3 schwenkbar um die zweite Schwenkachse 4 gelagert ist. Die beiden Schwenkachse 2, 4 stehen winkelig zueinander, schneiden sich aber nicht. Grundsätzlich ist es auch möglich, dass sich beide Achsen 2, 4 schneiden. Die Verdrehung um die erste Achse 2 erfolgt über einen Antrieb 5, der als Elektromotor ausgebildet ist und über ein Getriebe 10 in Gestalt eines Planetengetriebes und eine Kupplung 11 ein Abtriebselement 12 antreibt. Das Abtriebselement 12 ist als eine Schnecke ausgebildet, die um eine Drehachse 7 dreht. Beiderseits des Abtriebselementes 12 sind Anlaufscheiben und Lagerteile angeordnet, um das Abtriebselement 12 in dem Chassis 100 zu lagern. Das Abtriebselement 12 greift mit seiner Verzahnung in ein Schneckenrad als Antriebselement 17 ein und verdreht dieses in die eine oder andere Richtung um die Schwenkachse 2, je nach Drehrichtung des Elektromotors 5. Das Antriebselement 17 ist wiederum mit einem Kopplungselement 18 reversibel formschlüssig verbunden, was später näher erläutert wird. Dazu sind Federelemente 21 oberhalb des Kopplungselementes angeordnet, die sich an einem Vorsprung eines Trägers 19 abstützen. Der Träger 19 wiederum ist drehstarr mit dem Halter 24 gekoppelt und verschwenkt das Fingerelement 3, wenn der Träger 19 um die erste Schwenkachse 2 verdreht wird.

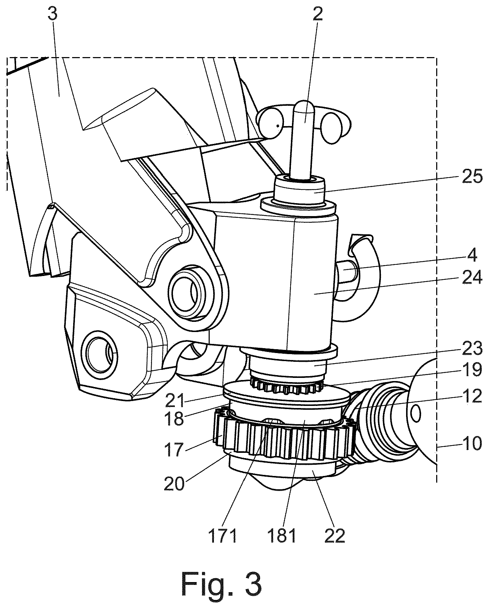

In der Figur 3 ist ein freigeschnittener Aufbau der Komponenten der Figur 2 in einer anderen Perspektive dargestellt. Wird die Schnecke 2 angetrieben, verdreht das Antriebselement 17 um die Schwenkachse 2. Das Antriebselement 17 stützt sich unten auf einem Spannelement 20 ab, unterhalb des Spannelementes 20 ist ein Magnetring 22 angeordnet, der als Signalgeber für einen nicht dargestellten Positionssensor in dem Chassis 100 dient. Das Antriebselement 17 weist an der oberen Stirnseite Rastelemente 171 oder Formschlusselemente auf, die über den Umfang verteilt an der Stirnseite angeordnet sind. Die Verteilung erfolgt vorteilhafterweise gleichmäßig über den Umfang. Oberhalb des Antriebselementes 17 steht das Kopplungselement 18 formschlüssig in Verbindung mit den Rastelementen 171, da die Unterseite des Kopplungselementes 18 korrespondierend zu der Oberseite des Antriebselementes 17 ausgebildet ist.

Das Kopplungselement 18 ist axial entlang der Längserstreckung der ersten Schwenkachse 2 an dem Träger 19 verschiebbar gelagert. Eine Verschiebung wird durch das Federelement 21 in Gestalt eines Tellerfeder-Paketes behindert. An der Innenseite des Kopplungselementes 18 ist eine Innenverzahnung ausgebildet, die korrespondierend zu einer Außenverzahnung an dem Träger 19 ausgebildet ist. Die Innenverzahnung des Kopplungselementes 18 greift in die Außenverzahnung des Trägers 19 ein und bewirkt bei einer Verdrehung des Antriebselementes 17, dass der Träger 19 um die erste Schwenkachse 2 verlagert wird. Der Träger 19 ist drehmomentübertragend oder drehstarr in dem Halter 24 gelagert, der wiederum zwei Lagerelemente 23, 25 aufweist, mit denen der Halter 24 in dem Chassis 100 gelagert ist. An dem Halter 24 ist die zweite Schwenkachse 4 ausgebildet, um die das Fingerelement 3 zum Greifen palmar verschwenkt werden kann.

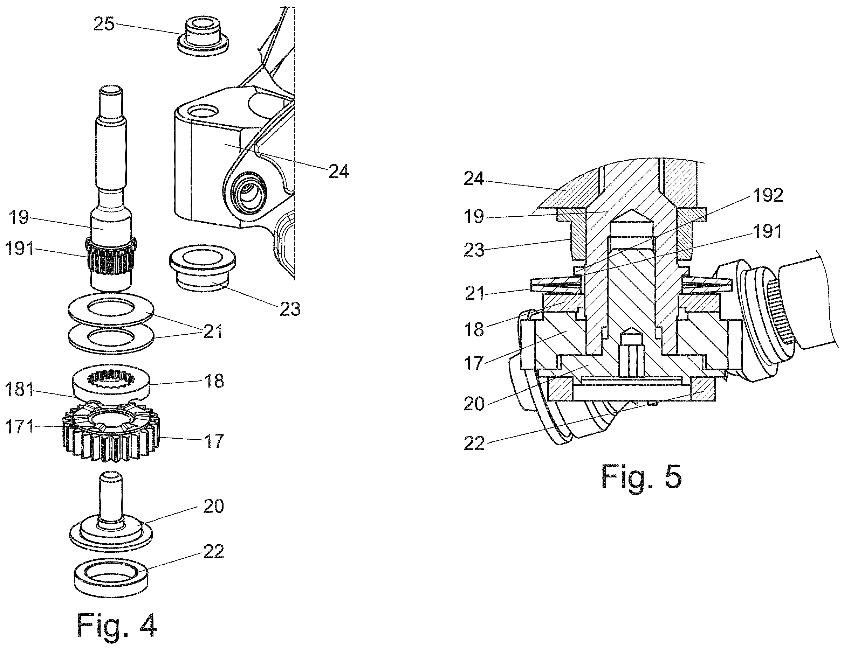

Der Figur 3 und besser der Figur 4 ist zu entnehmen, dass die Rastelemente 171 an dem Antriebselement 17 in beiden Umfangsrichtungen mit Anlauframpen oder Schrägen ausgebildet ist und die korrespondierenden Rastelemente 181 des Kopplungselementes 18 ebenfalls Anlauframpen oder Schrägen aufweisen. Bei einem aktivierten motorischen Antrieb 5 dreht sich das Abtriebselement 12 um die Drehachse 7 und treibt das Antriebselement 17 an. Das ansonsten frei drehbar an dem Träger 19 gelagerte Antriebselement 17 ist über die Rastelemente 171 und 181

Drehmomente übertragend mit dem Kopplungselement 18 verbunden. Das Kopplungselement 18 als Rastscheibe ist drehstarr über eine Verzahnung 191 mit dem Träger 19 gekoppelt, sodass eine Drehung des Antriebselementes 17 zu einer Drehung des Trägers 19 führt. Aufgrund der drehstarren Lagerung des Halters 24 an dem Träger 19 wird auch das Fingerelement 3 mitsamt dem Halter 24 um die erste Schwenkachse 2 verschwenkt. Sobald ein Widerstand gegen eine Verschwenkung um die erste Schwenkachse 2 auftritt, beispielsweise weil eine Endposition erreicht ist oder der Prothesenfinger 101 gegen einen Gegenstand gedrückt wird, kann es zu einer Relativbewegung zwischen dem Antriebselement 17 und dem Kopplungselement 18 kommen. Aufgrund der schrägen Ausgestaltung der Rastelemente 171, 181 ist eine Verdrehung bei gleichzeitiger axialer Verlagerung des Kopplungselementes 18 nach oben entlang der Längserstreckung der ersten Schwenkachse 2 möglich. Dies erfolgt gegen die Vorspannkraft, die durch die Federelemente 21 aufgebracht werden. Sofern das aufgebrachte Drehmoment zu groß ist, um von der formschlüssigen und reibschlüssigen Verbindung zwischen dem Antriebselement 17 und dem Kopplungselement 18 auf den Träger 19 übertragen zu werden, wird das Kopplungselement 18 soweit angehoben, dass dessen Unterseite auf der Oberseite des Rastelementes 171 des Antriebselementes 17 aufliegt und durchrutscht. Auf diese Weise wird beispielsweise eine Überlastsicherung bereitgestellt.

Um eine passive Verstellung des Fingerelementes 3 bzw. des Prothesenfingers um die erste Schwenkachse 2 zu bewirken, wird ein Drehmoment um die erste Schwenkachse 2 über den Halter 24 oder das Fingerelement 3 aufgebracht. Der Antriebsstrang von dem motorischen Antrieb 5 über das Abtriebselement 12 zu dem Antriebselement 17 ist selbsthemmend ausgebildet, sodass bei einer Drehmomentbeaufschlagung das Antriebselement 17 das Abtriebselement 12 nicht antreibt. Der Halter 24 mit dem Träger 19 verdreht dann relativ zu dem Antriebselement 17. Aufgrund der formschlüssigen und drehstarren Kopplung zwischen dem Träger 19 und dem Kopplungselement 18 führt dies zu einer Axialverlagerung des Kopplungselementes 18 nach oben gegen die Vorspannung der Federelemente 21, bis eine nächste Rastposition erreicht ist.

Über die Federelemente 21 wird die kraftschlüssige und formschlüssige Verbindung des Antriebselementes 17 und des Kopplungselementes 18 bis zu einem Grenzmoments sichergestellt. Oberhalb des Grenzmomentes verschiebt sich das Kopplungselement 18 oder die Rastscheibe axial auf dem Träger 19 entlang der Längserstreckung der ersten Schwenkachse 2, bis die maximale Höhe der Rastelemente 171 erreicht ist. Bei einem weiterhin aufgebrachten Drehmoment bewegt sich das Kopplungselement 18 zusammen mit dem Träger 19 und dem daran befestigten Halter 24 und dem Prothesenfinger 101 bis zur nächsten Rastposition.

**In** der Figur 4 ist zu erkennen, dass insgesamt sechs Rastelemente 171 bzw. Rasten an der oberen Stirnseite des Antriebselementes 17 angeordnet oder ausgebildet sind. Somit kann der Träger 19 mit dem Halter 24 und dem Prothesenfinger in sechs Rastpositionen verlagert werden, da auch sechs Ausnehmungen an der Unterseite des Kopplungselementes 18 vorhanden sind. Bei einer abweichenden Teilung oder bei einer größeren oder geringeren Anzahl von Rastelementen 171, 181 oder Rasten sind unterschiedliche Anzahlen an Rastpositionen möglich. Der Figur 4 ist zu entnehmen, dass das Kopplungselement 18 eine Innenverzahnung aufweist, die korrespondierend zu einer Außenverzahnungen 191 des Trägers 19 ausgebildet ist. Die Federelemente 21 stützen sich auf der Oberseite des Kopplungselementes 18 einerseits und an einem Absatz 192 der Außenverzahnungen 191 andererseits an dem Träger 19 ab und bewirken im montierten Zustand eine Vorspannung des Kopplungselementes 18 in Richtung auf das Antriebselement 17.

In der Figur 5 ist in einer Schnittdarstellung der montierte Zustand gemäß Figur 3 zu erkennen. Das Spannelement 20 weist ein Außengewinde auf, das in ein Innengewinde innerhalb des Trägers 19 eingeschraubt ist. Über das Spannelement 20 wird ein axiales Auflager für das frei um den Außenumfang des Trägers 19 drehbare Antriebselement 17 bereitgestellt. **In** dem Außenumfang des Spannelementes 20 ist ein Magnetring 22 angeordnet, der als Marker oder als Positionierhilfe dient, um eine Orientierung des Magnetfeldes und damit die tatsächliche Position des Prothesenfingers 101 relativ zu dem Chassis 100 festzustellen. Das Kopplungselement 18 oberhalb des Antriebselementes 17 wird über die beiden Tellerfedern des Federelementes 21, die an dem Absatz 192 der Außenverzahnungen 191 anliegen, gegenüber dem Antriebselement 17 vorgespannt. Der Halter 24 ist eingespannt oder aufgeschraubt oder auf andere Arten und Weisen an dem Träger 19 oberhalb der Verzahnung 191 drehfest gelagert.

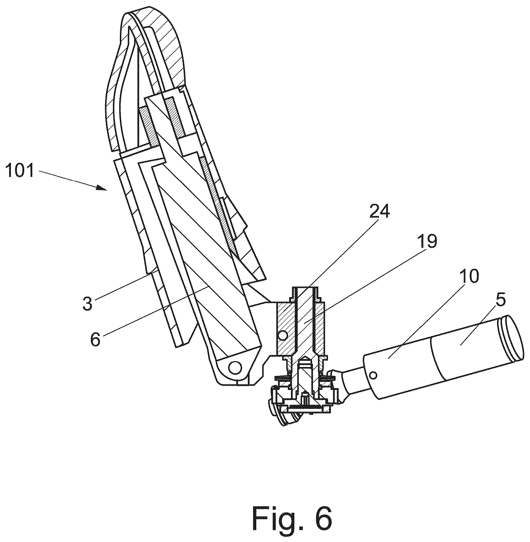

Die Figur 6 zeigte einen Schnitt durch den Prothesenfinger 101 mit einem innerhalb des Fingerelementes 3 angeordnetem Antrieb 6 zur Betätigung und Verschwenkung des Fingerelementes 3 um die zweite Schwenkachse 4, sodass ein Öffnen und ein Schließen des Daumens unabhängig von einer Abduktion oder Adduktion des Daumens um die erste Schwenkachse 2 erfolgen kann.

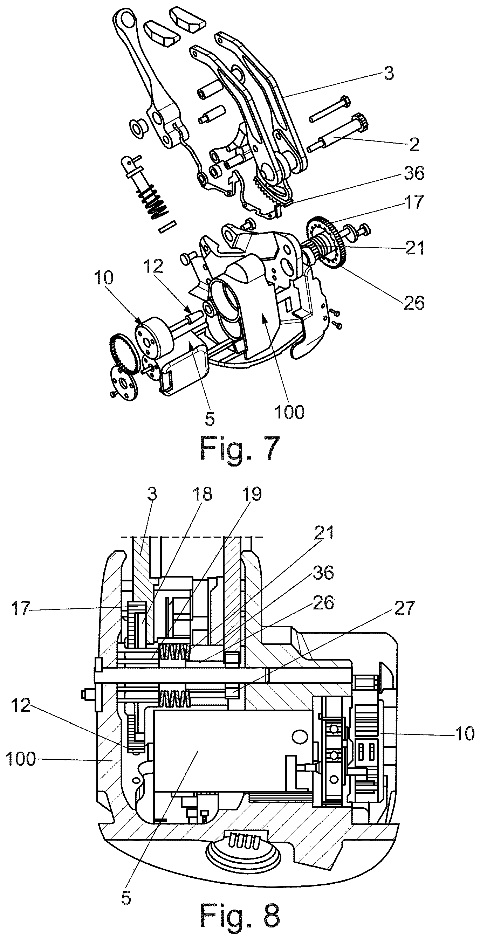

In der Figur 7 ist eine Variante dargestellt, bei der an dem Chassis 100 das Fingerelement 3 um eine erste Schwenkachse 2 schwenkbar gelagert ist. Innerhalb des Chassis 100 ist ein motorischer Antrieb 5 gelagert und über ein Getriebe 10 mit einem Abtriebselement 12 versehen. Das Abtriebselement 12 ist ein Zahnrad auf einer Welle, das mit dem Antriebselement 17 in Eingriff steht. Der grundsätzliche Aufbau des Antriebselementes 17 mit dem Kopplungselement 18, das in dieser Darstellung nicht zu erkennen ist, mit dem Träger 19 sowie dem Federelement 21 entspricht dem Aufbau der Ausführungsform gemäß der Figuren 2 bis 6. An dem Träger 19 ist ein Zahnrad 26 drehfest montiert und greift in ein Zahnradsegment 36 ein, das an dem Fingerelement 30 angeordnet, ausgebildet oder befestigt ist. Über das Zahnradsegment 36 wird bei einer Verdrehung des Zahnrades 26 das Fingerelement 3 um die erste Schwenkachse 2 an dem Chassis 100 in die eine oder andere Richtung bewegt.

In der Figur 8 ist in einer Schnittdarstellung aus einer anderen Perspektive das Chassis 100 mit dem darin angeordneten motorischen Antrieb 5 und dem Getriebe 10 dargestellt, über das das Abtriebselement 12 angetrieben wird. Das Abtriebselement 12 als Zahnrad greift in das Antriebselement 17 ein, das drehbar an dem Träger 19 gelagert ist. Das Kopplungselement 18 mit stirnseitigen, nicht dargestellten Rastelementen wird gegen das Antriebselement 17 durch das Federelement 21 gedrückt. Das Federelement 21 besteht aus mehreren Tellerfedern oder Tellerfederpaketen und belastet das Kopplungselement 18 in Längserstreckung oder in axialer Richtung in Richtung auf das Antriebselement 17. Das Kopplungselement 18 ist drehstarr und axial verschieblich an dem Träger 19 gelagert, der konkrete Aufbau wird später erläutert. Das Federelement 21 stützt sich auf der gegenüberliegenden Seite an dem Zahnrad 26 ab, das ebenfalls drehfest an dem Träger 19 gelagert und über eine Mutter 27 axial gesichert ist. Über die Mutter 27 lässt sich die Federvorspannung des Federelementes 21 einstellen. Das Zahnrad 26 kämmt das Zahnradsegment 36, das wiederum mit dem Fingerelement 3 drehmomentübertragend oder drehstarr gekoppelt ist, sodass eine Verdrehung des Zahnrades 26 zu einer Verschwenkung des Fingerelementes 3 um die Schwenkachse 2 führt.

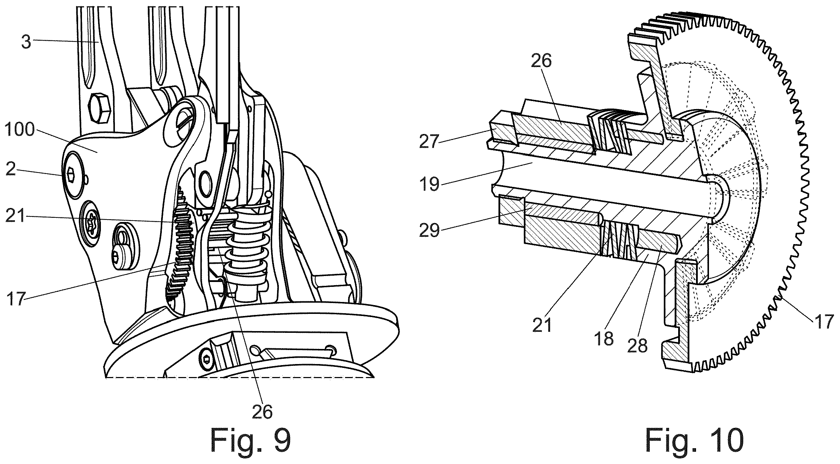

In der Figur 9 ist eine perspektivische Detailansicht des Fingerelementes 3 an dem Chassis 100 gezeigt. Das Fingerelement 3 ist um die erste Schwenkachse 2 an dem Chassis 100 gelagert. Innerhalb des Chassis 100 sind ebenfalls der Motor und das Getriebe gelagert. Zudem ist das Antriebselement 17 zu erkennen, das über das nicht zu erkennende Kopplungselement, das durch das Federelement 21 an das Antriebselement 17 angepresst wird, über den Träger 19 mit dem Zahnrad 26 gekoppelt ist, das wiederum das Fingerelement 3 antreibt.

In der Figur 10 ist in einer Schnittdarstellung der Aufbau der Komponenten mit dem Träger 19 dargestellt. An dem Träger 19 ist das Antriebselement 17 drehbar gelagert. Das Kopplungselement 18 ist mit einer Stirnverzahnung versehen und axial verschieblich und drehmomentübertragend an dem Träger 19 gelagert. Die drehstarre oder drehmomentübertragende Lagerung erfolgt über Stifte 28, die in korrespondierende Ausnehmungen in dem Träger 19 und der Bohrung des Kopplungselementes 18 angeordnet sind. Die Federelemente oder das Federelement 21 drückt das Kopplungselement 18 in Längserstreckung der Stifte 28 auf das Antriebselement 17, das sich an einem Absatz an dem Träger 19 abstützt.

Auf der anderen Seite des Federelementes 21 ist das Zahnrad 26 ebenfalls axial verschieblich und drehstarr über Stifte 29 fixiert. Eine Axialsicherung erfolgt über die Mutter 27. Die Stifte 29 sind in Ausnehmungen an der Außenseite des Trägers 19 bzw. der Innenseite des Zahnrades 26 ausgebildet.

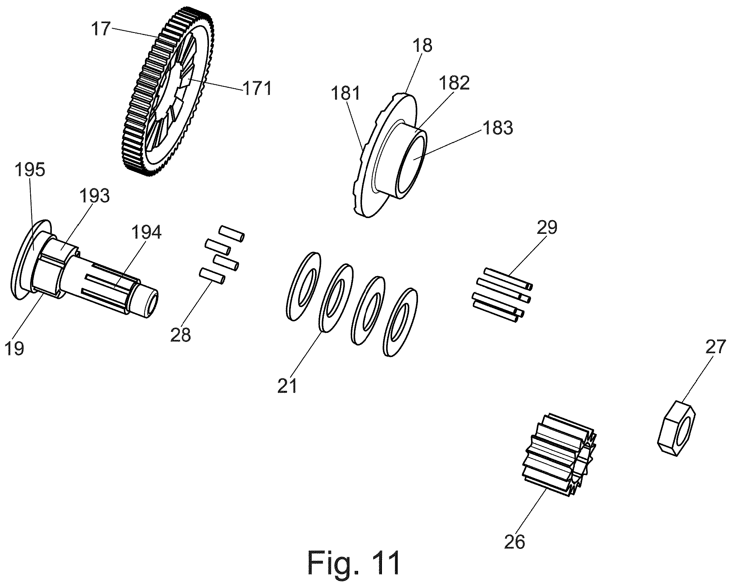

In der Figur 11 sind die einzelnen Komponenten der Figur 10 in einer Explosionsdarstellung gezeigt. An dem Träger 19 ist das Antriebselement 17 frei drehbar an einem unteren Absatz gelagert. Auf der dem Absatz 195 gegenüberliegenden Seite, die dem Kopplungselement 18 gegenüberliegt, sind Ausnehmungen bzw. Vorsprünge in Gestalt von Rastelementen 171 ausgebildet, in die korrespondierende Ausnehmungen 181 bzw. Vorsprünge an der Stirnseite des Kopplungselementes 18 eingreifen. Das Kopplungselement 18 weist auf der Innenseite eines Flansches 182 Ausnehmungen 183 auf, in die die Stifte 28 eingreifen. Korrespondierende Ausnehmungen 193 sind an der Außenseite des Trägers 19 ausgebildet, vorliegend vier Ausnehmungen 193, sodass sich eine axial verschiebliche, drehfest Lagerung des Kopplungselementes 18 an dem Träger 19 ergibt, entsprechend der Verzahnung in dem anderen Ausführungsbeispiel. Die Tellerfedern des Federelementes 21 stützen sich auf der Rückseite der Rastelemente 181 ab und werden auf der gegenüberliegenden Seite durch das Zahnrad 26 abgestützt, das in axialer Richtung an einem Gewinde über die Mutter 27 gesichert ist. Korrespondierend zu den Stiften 28 für das Kopplungselement 18 ist das Zahnrad 26 über sechs Stifte 29, in Ausnehmungen 194 an dem Träger 19 und an der Innenseite des Zahnrades 26 drehstarr und axial verschieblich befestigt. Das Zahnrad 26 greift in ein Zahnradsegment oder ein anderes Zahnrad zum Antrieb des Fingerelementes 3 ein.

## Patentansprüche

1. Prothesenfinger, mit einem motorischen Antrieb (5), über den der Prothesenfinger (101) relativ zu einem Chassis (100) um eine erste Schwenkachse (2) verschwenkbar ist, mit einem Fingerelement (3), das um die erste Schwenkachse (2) verschwenkbar an dem Chassis (100) gelagert ist und mit einem Träger (19), der mit dem Fingerelement (3) gekoppelt ist, an dem Träger (19) ist ein Antriebselement (17) gelagert, das mit dem motorischen Antrieb (5) koppelbar ist und drehmomentübertragend mit dem Träger (19) gekoppelt ist, **dadurch gekennzeichnet, dass** das Antriebselement (17) über ein Kopplungselement (18) mit dem Träger (19) drehmomentübertragend gekoppelt ist, wobei das Kopplungselement (18) axial verschieblich und in Richtung auf das Antriebselement (17) elastisch vorgespannt an dem Träger (19) gelagert ist.

2. Prothesenfinger nach Anspruch 1, **dadurch gekennzeichnet, dass** das Antriebselement (17) als Zahnrad oder Zahnradsegment ausgebildet ist und stirnseitig Rastelemente (171) und/oder Reibbereiche aufweist und das Kopplungselement (18) als Rastscheibe mit korrespondierend ausgebildeten Rastelementen (181) und/oder Reibbereichen ausgebildet ist

3. Prothesenfinger nach Anspruch 2, **dadurch gekennzeichnet, dass** die Rastelemente (171, 181) in zumindest einer Umfangsrichtung geneigt oder abgeschrägt ausgebildet sind.

4. Prothesenfinger nach Anspruch 2 oder 3, **dadurch gekennzeichnet, dass** die Rastelemente (171) an dem Antriebselement (17) sowie die korrespondierend ausgebildeten Rastelemente (181) an dem Kopplungselement (18) als Planverzahnung mit schraubflächenförmigen Zahnflanken ausgeführt sind.

5. Prothesenfinger nach einem der voranstehenden Ansprüche, **dadurch gekennzeichnet, dass** das Kopplungselement (18) formschlüssig an dem Träger (19) gelagert ist.

6. Prothesenfinger nach einem der voranstehenden Ansprüche, **dadurch gekennzeichnet, dass** der Träger (19) verschwenkbar um die erste Schwenkachse (2) gelagert und das Fingerelement (3) drehfest mit dem Träger (19) gekoppelt ist.

7. Prothesenfinger nach einem der Ansprüche 1 bis 5, **dadurch gekennzeichnet, dass** das Fingerelement (3) verschwenkbar an dem Träger (19) gelagert ist.

8. Prothesenfinger nach Anspruch 7, **dadurch gekennzeichnet, dass** das Fingerelement (3) verschwenkbar um die erste Schwenkachse (2) gelagert ist.

9. Prothesenfinger nach Anspruch 7 oder 8, **dadurch gekennzeichnet, dass** das Fingerelement (3) über ein Zahnrad oder Zahnradsegment mit dem Träger (19) gekoppelt ist.

10. Prothesenfinger nach einem der voranstehenden Ansprüche, **dadurch gekennzeichnet, dass** das Fingerelement (3) an einem Halter (24) schwenkbar um eine zweite Schwenkachse (4) gelagert und der Halter (24) an dem Träger (19) befestigt ist.

11. Prothesenfinger nach Anspruch 10, **dadurch gekennzeichnet, dass** das Fingerelement (3) einen Antrieb (6) aufweist und motorisch verschwenkbar an dem Halter (24) gelagert ist.

12. Prothesenfinger nach einem der Ansprüche 10 oder 11, **dadurch gekennzeichnet, dass** die Schwenkachsen (2, 4) sich kreuzend ausgerichtet sind.

13. Prothesenfinger nach einem der voranstehenden Ansprüche, **dadurch gekennzeichnet, dass** das Antriebselement (17) frei drehbar an dem Träger (19) gelagert ist.

14. Prothesenfinger nach einem der voranstehenden Ansprüche, **dadurch gekennzeichnet, dass** das Kopplungselement (18) in Richtung auf das Antriebselement (17) mit einer Vorspannkraft belastet ist, die eine Entkopplung des Kupplungselementes bei einem vorgegebenen Drehmoment zulässt.

15. Prothesenfinger nach einem der voranstehenden Ansprüche, **dadurch gekennzeichnet, dass** dem Träger (19) ein Positionssensor oder Marker zugeordnet ist, über den die Stellung des Trägers (19) relativ zu dem Chassis (100) erfasst wird.

16. Prothesenhand mit einem Chassis (100) und einem Prothesenfinger (101) nach einem der voranstehenden Ansprüche.

17. Prothesenhand nach Anspruch 16, **dadurch gekennzeichnet, dass** der motorische Antrieb (5) als Elektromotor ausgebildet ist, der ein Abtriebselement (12) antreibt, das mit dem Antriebselement (17) in Eingriff steht.

18. Prothesenhand nach Anspruch 17, **dadurch gekennzeichnet, dass** der motorische Antrieb (5) über ein Getriebe (10) und/oder eine Kupplung (11) mit dem Antriebselement (17) gekoppelt ist.

## Claims

1. A prosthetic finger with a motor drive (5) via which the prosthetic finger (101) is pivotable relative to a chassis (100) about a first pivot axis (2), with a finger element (3) which is mounted on the chassis (100) pivotably about the first pivot axis (2) and with a carrier (19) which is coupled to the finger element (3), on said carrier (19) a drive element (17) is mounted which can be coupled to the motor drive (5) and is coupled to the carrier (19) in a torque-transmitting manner,
**characterized in that** the drive element (17) is coupled to the carrier (19) in a torque-transmitting manner via a coupling element (18), wherein the coupling element (18) is mounted on the carrier (19) to be axially displaceable and elastically pretensioned in the direction of the drive element (17).

2. The prosthetic finger as claimed in claim 1, **characterized in that** the drive element (17) is designed as a toothed wheel or toothed wheel segment and has latching elements (171) and/or friction regions at a front end, and the coupling element (18) is designed as a latching disk with correspondingly designed latching elements (181) and/or friction regions.

3. The prosthetic finger as claimed in claim 2, **characterized in that** the latching elements (171, 181) are inclined or beveled in at least one circumferential direction.

4. The prosthetic finger as claimed in claim 2 or 3, **characterized in that** the latching elements (171) on the drive element (17) and the correspondingly designed latching elements (181) on the coupling element (18) are configured as a crown gear with helical tooth flanks.

5. The prosthetic finger as claimed in any one of the preceding claims,
**characterized in that** the coupling element (18) is mounted on the carrier (19) with a form fit.

6. The prosthetic finger as claimed in any one of the preceding claims,
**characterized in that** the carrier (19) is mounted pivotably about the first pivot axis (2), and the finger element (3) is coupled to the carrier (19) for conjoint rotation.

7. The prosthetic finger as claimed in any one of claims 1 to 5, **characterized in that** the finger element (3) is mounted pivotably on the carrier (19).

8. The prosthetic finger as claimed in claim 7, **characterized in that** the finger element (3) is mounted pivotably about the first pivot axis (2).

9. The prosthetic finger as claimed in claim 7 or 8, **characterized in that** the finger element (3) is coupled to the carrier (19) via a toothed wheel or toothed wheel segment.

10. The prosthetic finger as claimed in any one of the preceding claims,
**characterized in that** the finger element (3) is mounted on a holder (24) pivotably about a second pivot axis (4), and the holder (24) is secured on the carrier (19).

11. The prosthetic finger as claimed in claim 10, **characterized in that** the finger element (3) has a drive (6) and is mounted on the holder (24) pivotably by motor.

12. The prosthetic finger as claimed in either of claims 10 and 11, **characterized in that** the pivot axes (2, 4) are oriented crosswise.

13. The prosthetic finger as claimed in any one of the preceding claims,
**characterized in that** the drive element (17) is mounted freely rotatably on the carrier (19).

14. The prosthetic finger as claimed in any one of the preceding claims,
**characterized in that** the coupling element (18) is loaded in the direction of the drive element (17) with a pretensioning force that permits a decoupling of the coupling element at a predefined torque.

15. The prosthetic finger as claimed in any one of the preceding claims,
**characterized in that** the carrier (19) is assigned a position sensor or marker via which the position of the carrier (19) relative to the chassis (100) is detected.

16. A prosthetic hand having a chassis (100) and a prosthetic finger (101) as claimed in any one of the preceding claims.

17. The prosthetic hand as claimed in claim 16, **characterized in that** the motor drive (5) is designed as an electric motor, which drives an output element (12) that engages with the drive element (17).

18. The prosthetic hand as claimed in claim 17, **characterized in that** the motor drive (5) is coupled to the drive element (17) via a gearing (10) and/or a coupling (11).

## Revendications

1. Doigt prothétique comprenant un entraînement motorisé (5) permettant au doigt prothétique (101) de pivoter par rapport à un châssis (100) autour d'un premier axe de pivotement (2), un élément de doigt (3) monté sur le châssis (100) de manière à pouvoir pivoter autour du premier axe de pivotement (2), et un support (19) couplé à l'élément de doigt (3),
un élément d'entraînement (17) étant monté sur le support (19), qui peut être couplé à l'entraînement motorisé (5) et qui est couplé au support (19) de manière à transmettre le couple,
**caractérisé en ce que** l'élément d'entraînement (17) est couplé au support (19) par l'intermédiaire d'un élément de couplage (18) de manière à transmettre le couple, l'élément de couplage (18) étant monté sur le support (19) de manière à pouvoir se déplacer axialement et à être précontraint élastiquement en direction de l'élément d'entraînement (17).

2. Doigt prothétique selon la revendication 1,
**caractérisé en ce que** l'élément d'entraînement (17) est réalisé sous la forme d'une roue dentée ou d'un segment de roue dentée et présente sur sa face frontale des éléments d'encliquetage (171) et/ou des zones de frottement, et l'élément de couplage (18) est réalisé sous la forme d'un disque d'encliquetage muni d'éléments d'encliquetage (181) et/ou de zones de frottement réalisés de manière correspondante.

3. Doigt prothétique selon la revendication 2,
**caractérisé en ce que** les éléments d'encliquetage (171, 181) sont inclinés ou biseautés dans au moins une direction périphérique.

4. Doigt prothétique selon la revendication 2 ou 3,
**caractérisé en ce que** les éléments d'encliquetage (171) sur l'élément d'entraînement (17) ainsi que les éléments d'encliquetage (181) réalisés de manière correspondante sur l'élément de couplage (18) sont réalisés sous forme de denture plane ayant des flancs de dent en forme de surface à visser.

5. Doigt prothétique selon l'une des revendications précédentes,
**caractérisé en ce que** l'élément de couplage (18) est monté par complémentarité de forme sur le support (19).

6. Doigt prothétique selon l'une des revendications précédentes,
**caractérisé en ce que** le support (19) est monté de manière à pouvoir pivoter autour du premier axe de pivotement (2), et l'élément de doigt (3) est couplé solidairement en rotation au support (19).

7. Doigt prothétique selon l'une des revendications 1 à 5,
**caractérisé en ce que** l'élément de doigt (3) est monté sur le support (19) de manière à pouvoir pivoter.

8. Doigt prothétique selon la revendication 7,
**caractérisé en ce que** l'élément de doigt (3) est monté de manière à pouvoir pivoter autour du premier axe de pivotement (2).

9. Doigt prothétique selon la revendication 7 ou 8,
**caractérisé en ce que** l'élément de doigt (3) est couplé au support (19) par l'intermédiaire d'une roue dentée ou d'un segment de roue dentée.

10. Doigt prothétique selon l'une des revendications précédentes,
**caractérisé en ce que** l'élément de doigt (3) est monté sur un élément de maintien (24) de manière à pouvoir pivoter autour d'un deuxième axe de pivotement (4), et l'élément de maintien (24) est fixé au support (19).

11. Doigt prothétique selon la revendication 10,
**caractérisé en ce que** l'élément de doigt (3) présente un entraînement (6) et est monté sur l'élément de maintien (24) de manière à pouvoir pivoter par voie motrice.

12. Doigt prothétique selon l'une des revendications 10 ou 11,
**caractérisé en ce que** les axes de pivotement (2, 4) sont orientés de manière à se croiser.

13. Doigt prothétique selon l'une des revendications précédentes,
**caractérisé en ce que** l'élément d'entraînement (17) est monté sur le support (19) de manière à pouvoir tourner librement.

14. Doigt prothétique selon l'une des revendications précédentes,
**caractérisé en ce que** l'élément de couplage (18) est chargé en direction de l'élément d'entraînement (17) avec une force de précontrainte qui permet un découplage de l'élément de couplage à un couple prédéfini.

15. Doigt prothétique selon l'une des revendications précédentes,
**caractérisé en ce qu'**un capteur de position ou un marqueur est associé au support (19), au moyen duquel la position du support (19) par rapport au châssis (100) est détectée.

16. Main prothétique comprenant un châssis (100) et un doigt prothétique (101) selon l'une des revendications précédentes.

17. Main prothétique selon la revendication 16,
**caractérisée en ce que** l'entraînement motorisé (5) est conçu comme un moteur électrique qui entraîne un élément de sortie (12) qui est en prise avec l'élément d'entraînement (17).

18. Main prothétique selon la revendication 17,
**caractérisée en ce que** l'entraînement motorisé (5) est couplé à l'élément d'entraînement (17) par l'intermédiaire d'un engrenage (10) et/ou d'un accouplement (11).
